# EUROPEAN PATENT APPLICATION

(11) **EP 2 481 366 A1**
(43) Date of publication of application: **01.08.2012**
(21) Application number: 12152994.5
(22) Date of filing: 30.01.2012
(51) Int. Cl.: A61B 17/34, A61B 1/05

(54) **Insufflation needle with integrated image sensor**

(30) Priority: 31.01.2011 US 201161437778 P; 13.01.2012 US 201213349683
(71) Applicant: Tyco Healthcare Group LP, Mansfield, MA 02048 (US)
(72) Inventor: Stanley, Eric, Milford, Connecticut 06460 (US)
(74) Representative: Soames, Candida Jane

(57) **Abstract**

An insufflation apparatus (220) includes a housing defining a port for receipt of insufflation gases and an elongated sleeve (224) defining a longitudinal axis. The elongated sleeve has a proximal end and a distal end (225) and defines a sharpened tip (225). A stylet (226) is disposed within the elongated sleeve. The stylet is movable between an extended position, wherein the stylet extends beyond the tip of the sleeve, and a retracted position, to expose the sharpened tip for penetration through body tissue. At least one of the elongated sleeve and the stylet defines a passageway (229) in fluid communication with the port to direct the insufflation gases into a body cavity. An image sensor (240) is positioned on the elongated sleeve. The image sensor is adapted to receive an optical image of an area adjacent the distal end of the elongated sleeve and is configured to transmit the optical image for viewing by a clinician.

## Description

### CROSS REFERENCE TO RELATED APPLICATION

The present application claims the benefit of and priority to U.S. Provisional Application Serial No. 61/437,778, filed on January 31, 2011, the entire contents of which are incorporated herein by reference.

### BACKGROUND

### Technical Field

The present disclosure relates to surgical needles and, more particularly, to an insufflation, or pneumoperitoneum needle for inflating the peritoneal cavity that includes an integrated sensor for providing a video image of the contact point of the needle.

### Background of Related Art

Laparoscopic and endoscopic surgery has been widely accepted as the preferred surgical procedure for treatment of a variety of disorders that were formally treated with conventional surgical techniques.

In laparoscopic procedures, surgery is performed in the interior of the abdomen (e.g., the peritoneal cavity) through a small incision extending through the peritoneal cavity wall; in endoscopic procedures, surgery is performed in any hollow viscus of the body through narrow endoscopic tubes inserted through small entrance wounds in the skin.

In conjunction with laparoscopic surgery, pneumoperitoneum gases are generally introduced into the peritoneal cavity to expand the peritoneal cavity and raise the peritoneal cavity wall away from the vital organs therein. Thereafter, a trocar (e.g., a sharp pointed instrument) is inserted into a cannula assembly and used to puncture the inner lining of the peritoneal cavity. The trocar is then withdrawn and a laparoscopic surgical instrument is inserted through the cannula assembly to perform the desired surgery.

A conventional system used for introducing the pneumoperitoneum gases into the peritoneal cavity includes a pneumoperitoneum needle connected to a gas source via a flexible conduit. The pneumoperitoneum needle typically employed is a Veress-type needle which includes an elongated hollow outer sheath with a sharpened distal end for penetrating the inner lining of the peritoneal cavity. A spring-loaded blunt stylet is axially movable within the sheath and is distally biased so that the blunt end of the stylet retracts as the needle penetrates the inner lining and then advances to extend beyond the sharp end of the needle once the needle penetrates the inner lining of the peritoneal cavity. The pneumoperitoneum gas administering system also typically includes at least one volume flow regulator to control the rate of gas flow through the needle. Examples of such systems used for introducing pneumoperitoneum gases are disclosed 5,300,084, the entire contents of which are incorporated herein by reference.

### SUMMARY

In accordance with one embodiment of the present disclosure, an insufflation apparatus is provided. The insufflation apparatus includes a housing defining a port for receipt of insufflation gases. An elongated sleeve extends from the housing and defines a longitudinal axis. The elongated sleeve has a proximal end and a distal end defining a sharpened tip. A stylet is disposed within the elongated sleeve. The stylet is movable with respect to the sleeve between an extended position and a retracted position. In the extended position, the distal end of the stylet extends beyond the sharpened tip of the elongated sleeve. In the retracted position, the sharpened tip of the sleeve is exposed for penetration through body tissue. One (or both) of the elongated sleeve and the stylet defines a passageway in fluid communication with the port to direct the insufflation gases into a body cavity. An image sensor is positioned on the elongated sleeve. The image sensor is adapted to receive an optical image of an area adjacent the distal end of the elongated sleeve and is configured to transmit the optical image for viewing by a clinician.

In one embodiment, the image sensor is configured to receive an optical image of an area extending distally from and along the longitudinal axis of the elongated sleeve, i.e., the contact area of the sleeve. The optical image received by the image sensor may be transmitted to the external video display via wireless or wired communication. Further, the image sensor may be a CCD image sensor, a CMOS image sensor, or the like.

In another embodiment, the insufflation apparatus further includes a biasing member for biasing the stylet in the extended position.

In another embodiment, an illumination source may be provided for illuminating the contact area of the sharpened tip of the elongated sleeve, to allow for better visualization of the contact area. The illumination source may be a fiber optic bundle, an LED, or another suitable illumination source.

In yet another embodiment, the image sensor is configured to transmit the optical image to a control circuitry unit as an analog signal or, alternatively, as a digital signal. The control circuitry unit then transmits the signal to the external video display.

In still another embodiment, a protective cover, e.g., a lens or a clear epoxy, is disposed over the image sensor to protect and/or enhance the image sensor.

### BRIEF DESCRIPTION OF THE DRAWINGS

Various embodiments of the subject instrument are described herein with reference to the drawings wherein:

Fig. 1 is a side view of a pneumoperitoneum needle in accordance with one embodiment of the present disclosure;

Fig. 2 is a side, cross-sectional view of the pneumoperitoneum needle of Fig. 1;

Fig. 3 is a side, cross-sectional view of the pneumoperitoneum needle of Fig. 1 illustrating the insertion of the needle through body tissue;

Fig. 4 is a side, cross-sectional view of the pneumoperitoneum needle of Fig. 1 showing the needle positioned within an internal body cavity;

Fig. 5 is a perspective view of a surgical system in accordance with another embodiment of the present disclosure shown being inserted into body tissue;

Fig. 6 is an enlarged perspective view of an insufflation instrument and an access instrument of the surgical system of Fig. 5 shown being inserted into body tissue;

Fig. 7 is an enlarged perspective view of the insufflation instrument and access instrument of Fig. 6 shown accessing an internal body cavity;

Fig. 8 is a perspective of the surgical system of Fig. 5 illustrating use of the insufflation instrument to introduce fluids within the internal body cavity;

Fig. 9 is an enlarged side, cross-sectional view of a distal tip configuration of the pneumoperitoneum needle of Fig. 1 or the surgical system of Fig. 5 shown in a first position; and

Fig. 10 is an enlarged side, cross-sectional view of the distal tip configuration of the pneumoperitoneum needle of Fig. 1 or the surgical system of Fig. 5 shown in a second position.

### DETAILED DESCRIPTION

Embodiments of the presently disclosed surgical instruments will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

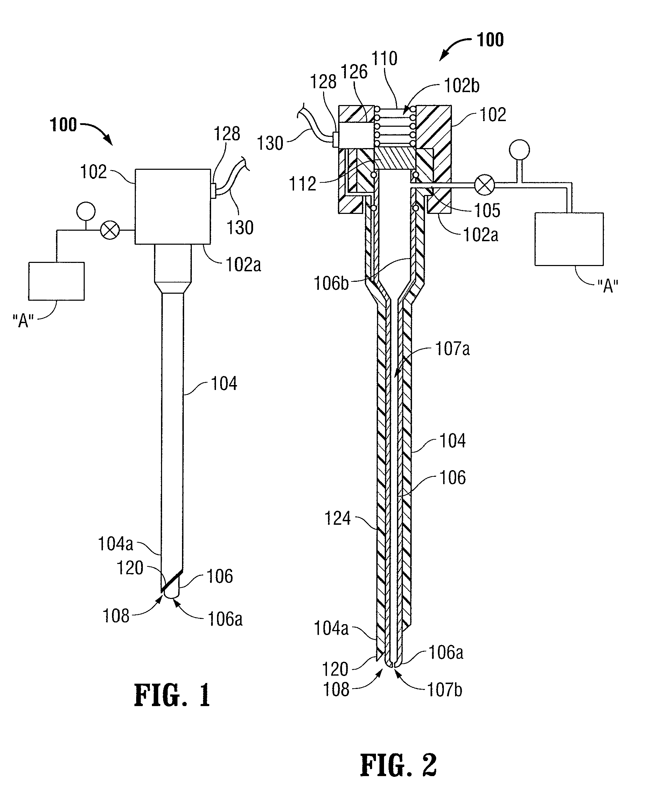

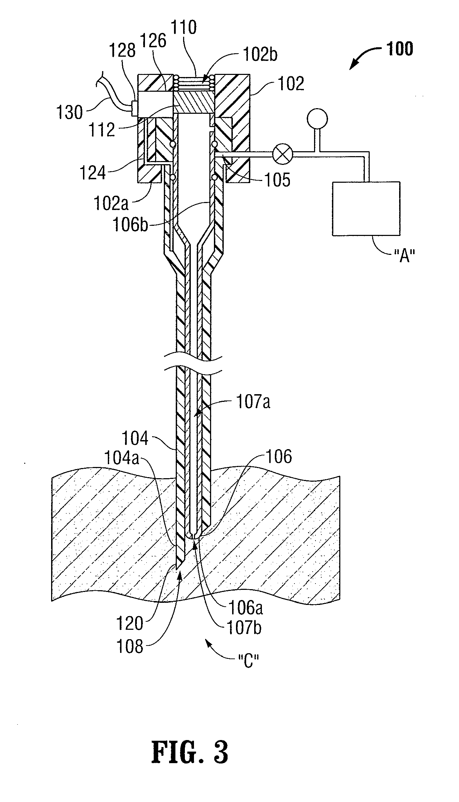

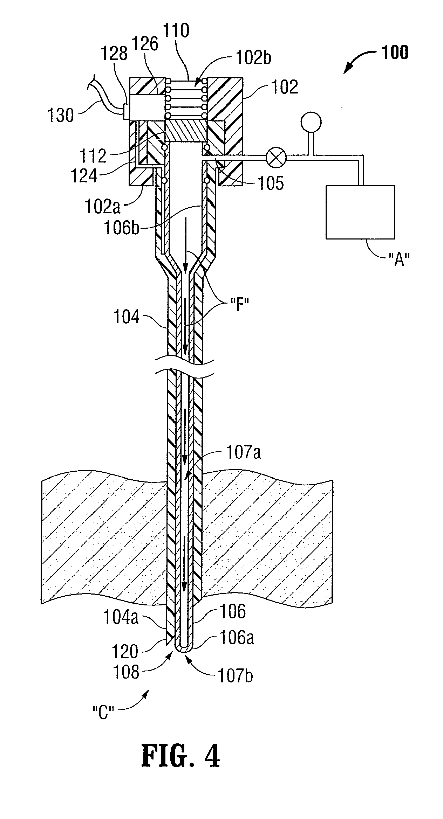

With reference to Figs. 1 and 2, an insufflation, or pneumoperitoneum needle in accordance with an embodiment of the present disclosure is generally indentified by reference numeral 100. Pneumoperitoneum needle 100 is similar to that of commonly-owned US Patent No. 7,618,399, the entire contents of which is hereby incorporated by reference herein. Needle 100 serves as a conduit between a source of pneumoperitoneum gas "A" (e.g., air, CO₂, etc) and the peritoneal cavity "C" (see Figs. 3 and 4), wherein the pneumoperitoneum gas "A" may enter and expand peritoneal cavity "C" to provide improved access to the internal organs therein during laparoscopic surgery. While the embodiments of the following disclosure will relate primarily to laparoscopic surgery, it is envisioned and within the scope of the present disclosure to apply the principles disclosed herein to numerous other surgical procedures, including, and not limited to, endoscopic, arthroscopic, and the like.

Pneumoperitoneum needle 100 includes a housing 102, an elongated hollow tubular body 104 operatively connected to a distal end 102a of housing 102, and a tubular rod 106 slidably received within tubular body 104. Pneumoperitoneum needle 100 is operatively connected to and, more specifically, is in fluid engagement with a source of pneumoperitoneum gas "A." Tubular body 104 includes a piercing edge or tip 108 formed at a distal end 104a thereof for penetrating the inner lining of the peritoneal cavity. Tubular body 104 further includes passage 105 formed therein for fluid communication with gas administering system "A."

Tubular rod 106 includes a blunt distal tip 106a, a proximal end portion 106b receivable in a cavity 102b formed in housing 102, and defines an elongate, longitudinally extending cavity 107a therethrough. Distal tip 106a of tubular rod 106 defines an opening 107b formed therein, which is, in this embodiment, is formed in a distally oriented direction, although it is envisioned that other orientations for opening 107b are possible.

With continued reference to Figs. 1-2, tubular rod 106 is sized such that distal tip 106a thereof extends beyond piercing edge 108 of tubular body 104 when tubular rod 106 is in a first or extended position, as shown in Fig. 2. Tubular rod 106 is adapted for reciprocal longitudinal movement from this first or extended position, as shown in Fig. 2, to a second or retracted position, as shown in Fig. 3, and is biased to the first or extended position under the influence of a coil spring 110. Spring 110 is disposed within cavity 102b of housing 102 such that one end of spring 122 is in contact with end plate 112 and the opposite end of spring 110 is in contact with an inner, distally oriented surface (not shown) of cavity 102b of housing 102.

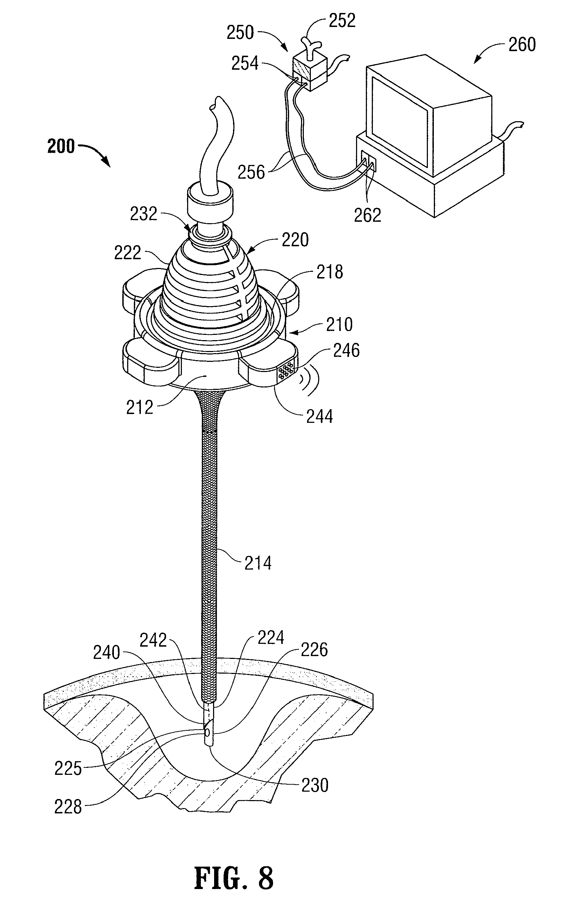

Pneumoperitoneum needle 100 further includes an integrated visualization sensor assembly 120 disposed at distal end 104a of tubular body 104, i.e., at the contact point of piercing edge 108 of tubular body 104, for providing a video image of the area extending distally from and in the direction of pneumoperitoneum needle 100. The sensor assembly 120, as will be described in greater detail below, includes a cable, or wire 122 (or bundle of wires) extending proximally through tubular body 104 from distal end 104a of tubular body 104 into housing 102. Cable 124 couples sensor assembly 120, disposed at distal end 104a of tubular body 104, to control circuitry 126, which is disposed within housing 102. Control circuitry 126 is coupled to a transmitter 128 for transmitting a signal received from the sensor assembly 120 (via cable 124) to an external video display 260 (see Fig. 8) via transmission cable 130. However, wireless transmission of the signal from the transmitter 128 to the video display 260 (see Fig. 8) is also contemplated. The components and operation of sensor assembly 120 will be described in greater detail below.

Turning now to Figs. 3-4, during use of pneumoperitoneum needle 100, when tip 108 of tubular body 104 is being inserted into body tissue, i.e., as tip 108 of tubular body 104 is pressed against the skin of the patient, distal tip 106a of tubular rod 106 is urged from the first position to the second position, i.e., into tubular body 104. When tubular rod 106 is in the second, or retracted position, pneumoperitoneum gas is prevented from entering cavity 107a and, thus, is prevented from passing through opening 107b and into the peritoneal cavity "C." During the advancement of pneumoperitoneum needle 100 through tissue and into the peritoneal cavity "C," visualization sensor assembly 120 provides the surgeon with a video image of the contact point of needle 100, allowing the surgeon to determine the position of tip 108 of tubular body 104 relative to surrounding tissue.

Once tip 108 of tubular body 104 completely penetrates the abdominal wall of the patient, distal tip 106a of tubular rod 106 is no longer substantially obstructed and, thus, is permitted to move back to the first, or extended position under the bias of coil spring 110. In this position, gas flows from the insufflation gas source "A," through lumen 107a of tubular rod 106, i.e., in the direction of arrows "F," to supply gas to peritoneal cavity "C." Further, in this position, tip 108 is protected, i.e., unexposed, due to the extended position of tubular rod 106, such that inadvertent puncture of tissue is inhibited.

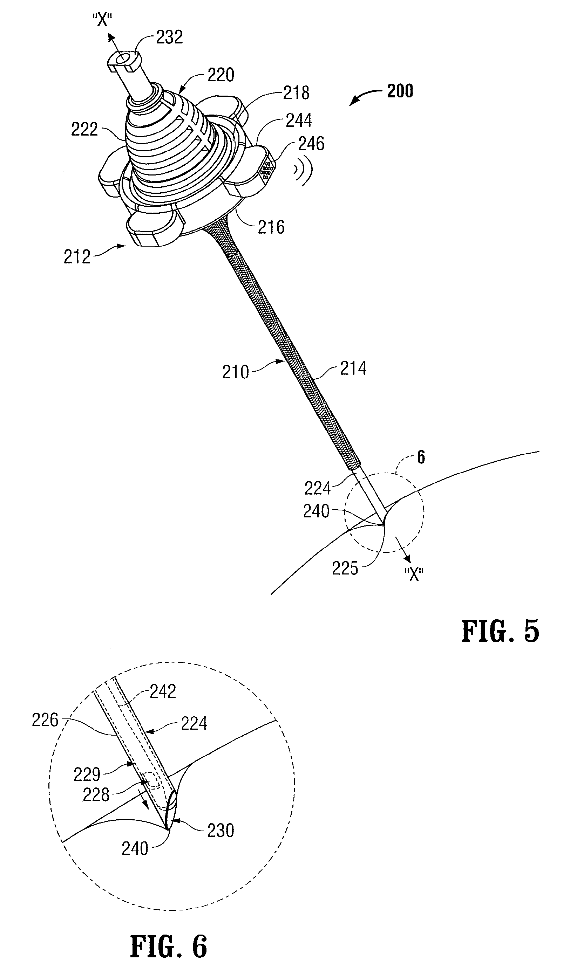

Referring now to Figs. 5-8, a surgical system for insufflating and permitting access to an underlying body cavity in accordance with the principles of the present disclosure is generally identified by reference numeral 200. Surgical system 200 is similar to that of commonly-owned US Patent No. 7,329,233, the entire contents of which are hereby incorporated by reference herein.

Surgical system 200 includes an access instrument 210 and an insufflation instrument 220 which is at least partially positionable within the access instrument 210. Access instrument 210 provides access through tissue and into an underlying body cavity, e.g., the abdominal or peritoneal cavity, while insufflation instrument 220 is used to introduce insufflation gases into the body cavity to expand the cavity to facilitate access to the organs and tissue therein.

Access instrument 210 generally includes an access housing 212 and elongate member 214 extending from the access housing 212. Access housing 212 and elongate member 214 define a longitudinal axis "X" which extends through and along the length of access instrument 210. Access housing 212 includes a base 216 and a hub 218 which at least partially resides within the base 216. Elongate member 214 of access instrument 210 extends distally from access housing 212 and defines a generally tubular shape.

With continued reference to Figs. 5-8, insufflation instrument 220 includes housing 222 and insufflation sleeve 224 extending distally from the housing 222. Housing 222 generally defines an oval or egg shape and includes a pair of locking tabs (not shown) extending radially outwardly from the outer surface of housing component for securing or locking insufflation instrument 220 within access instrument 210. Insufflation housing 222 further includes port 232 at a proximal end of housing 222 which connects to a supply of insufflation gas or gaseous media such as CO₂ gas as is known in the art.

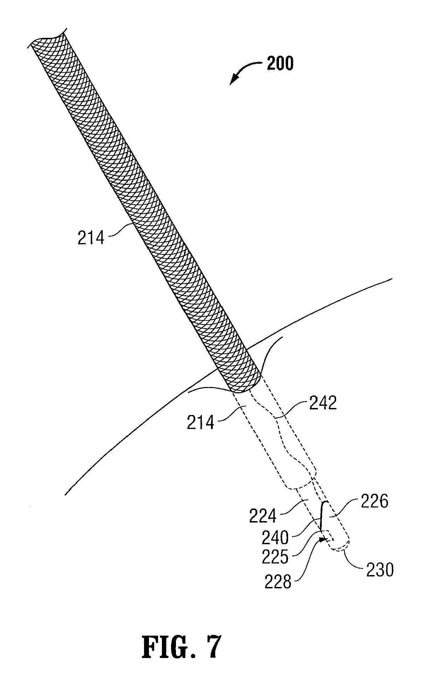

Insufflation sleeve 224 is securely mounted to insufflation housing 222 by conventional means. Insufflation sleeve 224 is generally tubular in shape and defines a sharpened distal end 225 (e.g., a beveled end) to assist in penetrating the body tissue. A stylet 226 is disposed within the interior of the sleeve 224 and includes apertures 228 disposed at a distal end thereof. Stylet 226 further defines a lumen 229 extending therethrough in communication with apertures 228 at the distal end thereof and in communication with port 232 at the proximal end thereof such that, upon activation, insufflation gas may flow through stylet 226 and into the internal body cavity through apertures 228. Further, stylet 226 may be biased toward a first, or extended position, as shown in Figs. 7-8, wherein stylet 226 protrudes, or extends distally from sleeve 224. Stylet 226 is moveable with respect to sleeve 224 from the first, or extended position to a second, or retracted position, as shown in Fig. 6, wherein stylet is completely disposed within sleeve 224, thereby exposing sharpened distal end 225 of sleeve 224.

Similar to pneumoperitoneum needle 100, discussed above, surgical system 200 further includes an integrated visualization sensor assembly 240 disposed at a distal end of insufflation sleeve 224, i.e., the contact point of insufflation sleeve 224, for providing a video image of the area extending distally from and in the direction of insufflation instrument 220. The sensor assembly 240, as will be described in greater detail below, includes a wire 242 (or bundle of wires) that extends from the distal end of insufflation sleeve 224 proximally into housing 222, ultimately coupling to a set of electrical contacts (not shown) positioned on an external surface of insufflation housing 222. The electrical contacts (not shown) are configured for electrical coupling with corresponding contacts (not shown) disposed on an inner surface of access housing 212 of access instrument 210 such that, upon the fixing, or locking of insufflation instrument 220 within access instrument 210, as described above, electrical communication between insufflation housing 222 and access housing 212 is established. The contacts (not shown) of access housing 212 of access instrument 210 are ultimately coupled to a control circuitry unit 244 of access housing 212 such that, upon the locking of insufflation instrument 220 within access instrument 210, sensor assembly 240 is communicable with control circuitry unit 244, and visa versa. Alternatively, any other suitable communication mechanism may be provided, e.g., control circuitry unit 244 may be disposed on insufflation instrument 220 such that the electrical contacts are not required, or the signal form the sensor assembly 240 may be communicated to the control circuitry 244 wirelessly.

With continued reference to Figs. 5-8, control circuitry unit 244 is coupled to a wireless transmitter 246 for wirelessly transmitting the signal received from the sensor assembly 240 (via wire(s) 242 and the electrical contacts) to a remotely positioned wireless receiver 250. The wireless receiver 250 is coupled to a video display 260, which is configured to display the wireless signal received from the wireless transmitter 246 as a video image. The components and operation of sensor assembly 240 will be described in greater detail below.

In use, as best shown in Figs. 6-8, insufflation instrument 220 is positioned within access instrument 210 and secured thereto. Insufflation instrument 220 is then applied against the patient's abdominal area wherein, upon contacting the tissue with blunt end 230 of stylet 226, the stylet 226 retracts from the first position to the second position to expose sharpened end 225 of insufflation sleeve 224.

The procedure is continued by applying force to insufflation instrument 220 such that sharpened end 225 of insufflation sleeve 224 penetrates the tissue to enter the abdominal cavity, as shown in Fig. 6. During the advancement of insufflation instrument 220 through tissue, visualization sensor assembly 240 provides the surgeon with a video image of the contact point of sharpened end 225 of insufflation sleeve 224, allowing the surgeon to determine the relative position of sharpened end 225 of insufflation sleeve 224 with respect to surrounding tissue.

Once the cavity is accessed, stylet 226 is free to move distally to the first, or extended position, as shown in Fig. 7. In this position, blunt end 230 of stylet 226 extends beyond sharpened end 225 of insufflation sleeve 224 to prevent puncture or laceration of internal abdominal structures. The gaseous supply is connected to port 232 to permit insufflation gases to flow through lumen 229 of stylet 226 and out apertures 228 to expand the peritoneal cavity. Upon achieving the desired pressure, insufflation instrument 220 may be removed from access instrument 210, leaving access instrument 210 within the abdominal cavity. Thereafter, access instrument 210 may be utilized as a conduit for insertion of instruments, scopes, etc. to perform the desired surgical task.

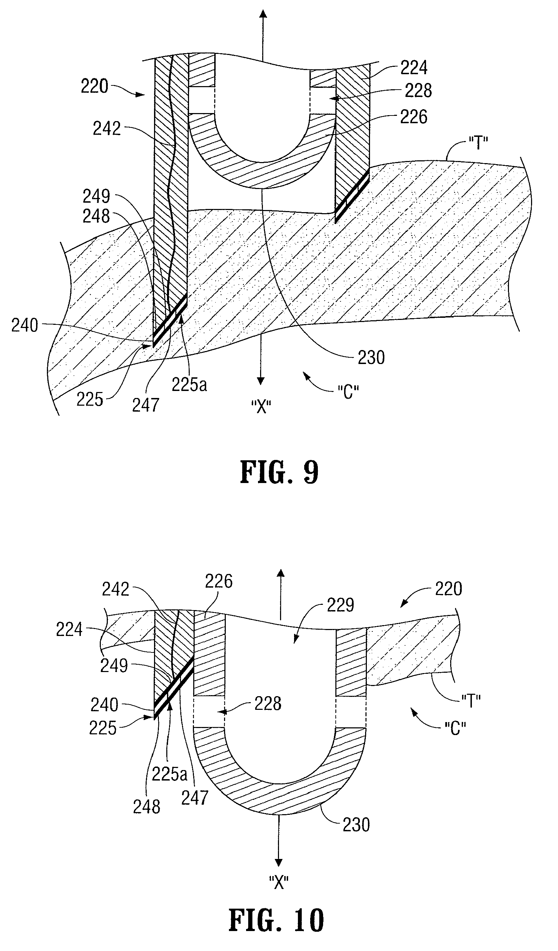

Referring now to Figs. 9 and 10, the components and operation of sensor assemblies 120, 240 (Figs. 1-4 and Figs. 5-8, respectively) will be described in detail. As shown in Figs. 9 and 10, sensor assembly 240 of surgical system 200 is disposed at the distal end of sleeve 224 of insufflation instrument 220. Sensor assembly 120 of pneumoperitoneum needle 100 is similarly positioned, i.e., is disposed in distal end 104a of tubular body 104 of pneumoperitoneum needle 100, and functions in a substantially similar manner as will be described below with regard to sensor assembly 240 of surgical system 200. Thus, only the difference between sensor assembly 120 of pneumoperitoneum needle 100 and sensor assembly 240 of surgical system 200 will be described below to avoid unnecessary repetition.

As best shown in Figs. 9 and 10, in conjunction with Fig. 8, and as mentioned above, surgical system 200 includes an integrated sensor assembly 240 disposed on a generally distally-facing surface 225a, i.e., the contact point, of sharpened distal tip 225 of sleeve 224. As discussed above, sensor assembly 240 may be configured to wirelessly transmit a signal, e.g., a digital image signal, to a wireless receiver 250, or, alternatively, may be configured for wired transmission of the digital image signal to the receiver 250, similar to sensor assembly 120 of pneumoperitoneum needle 100. The wireless receiver 250 is configured to decouple the signal and feed the signal to a video display 260 to display the signal as a video image. As can be appreciated, a surgical system 200 including an integrated sensor assembly 240 allows a surgeon as well as the surgical team to view a real-time image of the surgical site on a video display 260, without the need for additional incisions or larger incisions to allow cameras or sensors to be inserted into the body. More specifically, the sensor assembly 240, in conjunction with the video monitor 260, provides the surgeon with a real-time image of the contact area of sharpened distal tip 225 of insufflation instrument 220, e.g., the area extending distally from and in a similar direction as pointed distal tip 225 of sleeve 224, thereby allowing the surgeon to visually confirm the state of the insufflation sleeve 224, e.g., whether the sleeve 224 is in the retracted position or the extended position, and the relative position of the insufflation instrument 220 with respect to surrounding tissue. Such a feature helps prevent inadvertent damage, e.g., puncture, to internal body tissue. The sensor assembly 240 is also advantageous in that it is integral with, or disposed substantially within, the surgical system 200 and, thus, does not require altering the general dimensions or configuration of the surgical system 200 in order to accommodate the components of sensor assembly 240.

Continuing with reference to Figs. 8-10, sensor assembly 240 includes a protective cover 247, an illumination source 248, and an image sensor 249. The protective cover 247 may be a lens configured to project an optical image onto the image sensor 249, or may be a clear adhesive, epoxy, or other suitable cover configured to protect the sensor 249 from debris, fluids, and the like. In embodiments where a lens is provided, the lens may be configured to focus, magnify, or otherwise modify the optical image projected onto the image sensor 249.

The illumination source 248 may include a fiber optic bundle extending through the sleeve 224 and terminating at distal tip 225 thereof for illuminating the field of view. Alternatively, one or more LED's 248 may be positioned at the distal end 225 of sleeve 224 for illuminating the field of view, or an external illumination source (not shown) may be used for illumination purposes.

The image sensor 249 is configured to receive an optical image of the field of view, i.e., the area extending distally from and in the general direction of distal tip 225 of sleeve 224, and to convert the optical image into an electrical signal. The image sensor 249 may be a CCD image sensor, a CMOS image sensor, or any other suitable image sensor as is known in the art. Further, the image sensor 249 may be either a digital or an analog image sensor and, thus, may be configured to produce either a digital or an analog signal.

As shown in Figs. 9-10, the image sensor 249 is electrically coupled to insulated wire, or bundle of wires 242 extending from the image sensor assembly 240 proximally through sleeve 224 to insufflation housing 222. Bundle of wires 242 is configured to transmit the electrical signal produced by the image sensor 249 to the control circuitry unit 244, e.g., via the electrical contacts (not shown). Bundle of wires 242 may also be configured to transfer power to the image sensor 249 from a battery (not shown) disposed within control circuitry unit 244 of insufflation housing 222 or, alternatively, from an external power source (not shown), via either wired or wireless power transmission.

Control circuitry unit 244 includes a processing component and a wireless transmitter 246. More specifically, the signal produced by the image sensor 249 is communicated to the processing component of the control circuitry unit 244, which processes the signal, e.g., converts the signal from analog to digital or digital to analog, or modulates the signal. In one embodiment, for example, the image sensor 249 communicates an analog signal to the processing component which, in turn, synthesizes the signal with a carrier frequency, e.g., 2.4 GHz, and communicates the modulated signal to the wireless transmitter 246. Where the signal is a digital signal, the processing component may be configured to first convert the signal to analog before modulating the signal and transmitting the signal to the wireless transmitter 246. In another embodiment, for example, the image sensor 249 communicates a digital signal to the processing component, which digitally modulates the signal and communicates the signal to the wireless transmitter 246. If the signal from the image sensor 249 is analog, the processing component may be configured to digitize the signal before communicating the signal to the wireless transmitter 246.

The wireless transmitter 246 is configured to wirelessly transmit, or broadcast the processed signal to the wireless receiver 250. As mentioned above, in some embodiments, the signal is analog, or converted to analog, and modulated with a carrier frequency, e.g. 2.4 GHz, by the processing component of the control circuitry unit 244. Accordingly, the wireless transmitter 246 may be configured to broadcast the modulated analog signal to the wireless receiver 250. In other embodiments, where the signal is digital, or digitized, and modulated by the processing component, the wireless transmitter 246 may be configured according to a standard protocol, e.g., Bluetooth, Wi-Fi, or Zigbee. Alternatively, any other suitable configuration of wireless transmitter, standard or proprietary, may be used. Further, wireless transmitter 246 may include an antenna (not shown) extending therefrom to facilitate transmission of the signal to the wireless receiver 250. The antenna (not shown) may be configured as a low profile antenna protruding minimally from access housing 112, or may be internally disposed within access housing 112.

With continued reference to Figs. 8-10, the wireless transmitter 246 is configured to transmit the signal wirelessly to the wireless receiver 250. It is envisioned that the wireless receiver 250 also include an antenna 252 to facilitate reception of the signal from the wireless transmitter 246. It is further envisioned that the wireless transmitter 246 and wireless receiver 250 have a working range suitable for use in an operating room or other surgical setting. In other words, it is envisioned that the wireless transmitter 246 be capable of communication with the remote wireless receiver 250 throughout the entire surgical procedure, as the surgical system 200 is maneuvered during the course of the procedure.

The wireless receiver 250 may be a standard wireless receiver, e.g., a Bluetooth, Wi-Fi, Zigbee, or other off-the-shelf product according to the wireless transmitter 246, or alternatively, may be specifically configured according to the specifications of the wireless transmitter 246. In either embodiment, the wireless receiver 250 is configured to decouple, or demodulate, the signal and communicate the signal to the video monitor 260. The wireless receiver 250 may include standard electrical connections 254 such that the wireless receiver 250 may be coupled, e.g., via cables 256, to corresponding electrical connections 262 of any standard video monitor 260. The video monitor 260 displays the signal as a video image.

In embodiments where transmission of the image from the transmitter to the receiver is wired, e.g., in the embodiment of sensor assembly 120 of pneumoperitoneum needle 100 (Figs. 1-4), the functionality of the sensor assembly 120, control circuitry 126 and transmitter 128 is substantially similar to that of the wireless transmitter/receiver of sensor assembly 240 of surgical system 200 described above, except that the signal would be transmitted along a cable, or wire 130 that is coupled at a first end to the transmitter 128 and at a second end to the receiver.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:
1. An insufflation apparatus, which comprises a housing defining a port for receipt of insufflation gases; an elongated sleeve extending from the housing and defining a longitudinal axis, the elongated sleeve having a proximal end and a distal end, the distal end defining a sharpened tip; a stylet disposed within the elongated sleeve, the stylet being movable between an extended position wherein the distal end of the stylet extends beyond the sharpened tip of the elongated sleeve and a retracted position to expose the sharpened tip for penetration through body tissue; at least one of the elongated sleeve and the stylet defining a passageway in fluid communication with the port to direct the insufflation gases into a body cavity, and an image sensor positioned on the elongated sleeve, the image sensor adapted to receive an optical image of an area adjacent the distal end of the elongated sleeve and configured to transmit the optical image for viewing by a clinician.
2. The insufflation apparatus according to paragraph 1, wherein the image sensor is configured to receive an optical image of an area extending distally from and along the longitudinal axis of the elongated sleeve.
3. The insufflation apparatus according to paragraph 1, wherein the stylet defines a lumen in fluid communication with the port to direct the insufflation gases into a body cavity.
4. The insufflation apparatus according to paragraph 1, further comprising a biasing member for biasing the stylet in the extended position.
5. The insufflation apparatus according to paragraph 1, wherein the optical image is transmitted to the external video display through a wireless communication device.
6. The insufflation apparatus according to paragraph 1, wherein the image sensor includes one of a CCD image sensor and a CMOS image sensor.
7. The insufflation apparatus according to paragraph 1, further comprising an illumination source for illuminating the area distally adjacent and along the longitudinal axis of the elongated sleeve.
8. The insufflation apparatus according to paragraph 7, wherein the illumination source is one of a fiber optic bundle and an LED.
9. The insufflation apparatus according to paragraph 1, wherein the image sensor is configured to transmit one of an analog and a digital signal of the optical image to a control circuitry unit.
10. The insufflation apparatus according to paragraph 9, wherein the control circuitry unit transmits the one of an analog and a digital signal to the external video display.
11. The insufflation apparatus according to paragraph 1, further comprising a protective cover disposed over the image sensor.
12. The insufflation apparatus according to paragraph 11, wherein the protective cover is a lens.

## Claims

1. An insufflation apparatus, which comprises:
a housing defining a port for receipt of insufflation gases;
an elongated sleeve extending from the housing and defining a longitudinal axis, the elongated sleeve having a proximal end and a distal end, the distal end defining a sharpened tip;
a stylet disposed within the elongated sleeve, the stylet being movable between an extended position wherein the distal end of the stylet extends beyond the sharpened tip of the elongated sleeve and a retracted position to expose the sharpened tip for penetration through body tissue;
at least one of the elongated sleeve and the stylet defining a passageway in fluid communication with the port to direct the insufflation gases into a body cavity, and
an image sensor positioned on the elongated sleeve, the image sensor adapted to receive an optical image of an area adjacent the distal end of the elongated sleeve and configured to transmit the optical image for viewing by a clinician.

2. The insufflation apparatus according to claim 1, wherein the image sensor is configured to receive an optical image of an area extending distally from and along the longitudinal axis of the elongated sleeve.

3. The insufflation apparatus according to claim 1 or claim 2, wherein the stylet defines a lumen in fluid communication with the port to direct the insufflation gases into a body cavity.

4. The insufflation apparatus according to any preceding claim, further comprising a biasing member for biasing the stylet in the extended position.

5. The insufflation apparatus according to any preceding claim, wherein the optical image is transmitted to the external video display through a wireless communication device.

6. The insufflation apparatus according to any preceding claim, wherein the image sensor includes one of a CCD image sensor and a CMOS image sensor.

7. The insufflation apparatus according to any preceding claim, further comprising an illumination source for illuminating the area distally adjacent and along the longitudinal axis of the elongated sleeve.

8. The insufflation apparatus according to claim 7, wherein the illumination source is one of a fiber optic bundle and an LED.

9. The insufflation apparatus according to any preceding claim, wherein the image sensor is configured to transmit one of an analog and a digital signal of the optical image to a control circuitry unit.

10. The insufflation apparatus according to claim 9, wherein the control circuitry unit transmits the one of an analog and a digital signal to the external video display.

11. The insufflation apparatus according to any preceding claim, further comprising a protective cover disposed over the image sensor.

12. The insufflation apparatus according to claim 11, wherein the protective cover is a lens.
